(19) Europäisches Patentamt
European Patent Office
Office européen des brevets

(11) Publication number: 0 285 388 A2

(12) # EUROPEAN PATENT APPLICATION

(21) Application number: 88302821.9

(22) Date of filing: 30.03.88

(51) Int. Cl.[4]: A 61 K 7/08

(30) Priority: 01.04.87 US 33443 01.03.88 US 156510
10.04.87 US 36656

(43) Date of publication of application:
05.10.88 Bulletin 88/40

(84) Designated Contracting States:
AT BE CH DE FR GB GR IT LI LU NL SE

(71) Applicant: THE PROCTER & GAMBLE COMPANY
One Procter & Gamble Plaza
Cincinnati Ohio 45202 (US)

(72) Inventor: Grote, Mark Bernard
3416 Ferncroft Drive
Cincinnati Ohio 45211 (US)

Dzialo, Kathleen Brown
1505 Governor Terrace
Cincinnati Ohio 45215 (US)

Hutchinson, Neal Kevin
8510 Capricorn Drive
Montgomery Ohio 45249 (US)

(74) Representative: Brooks, Maxim Courtney et al
Procter & Gamble (NTC) Limited Whitley Road
Longbenton
Newcastle-upon-Tyne NE12 9TS (GB)

(54) **Shampoo compositions.**

(57) Lotion shampoos are disclosed which comprise a synthetic surfactant, an insoluble, nonvolatile silicone, a pyridinethione salt, a suspending agent and water.

EP 0 285 388 A2

## Description

# SHAMPOO COMPOSITIONS

## CROSS REFERENCE TO RELATED APPLICATIONS

This is a continuation-in-part application of my application Serial No. 033,443, filed April 1, 1987.

## TECHNICAL FIELD

The present invention is related to lotion antidandruff shampoos which contain a dispersed, nonvolatile silicone phase and a pyridinethione salt and are stabilized through the use of certain long chain materials.

## BACKGROUND OF THE INVENTION

Antidandruff shampoos are well known in the prior art and rely on a number of different agents for their antidandruff effectiveness. However shampooing with an antidandruff shampoo is done not only to relieve a dandruff condition but also to clean the hair.

Human hair becomes soiled due to its contact with the surrounding atmosphere and, to a greater extent, from sebum secreted by the head. The build-up of the sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled and generally unmanageable state. A variety of approaches have been developed to alleviate the after-shampoo problems. These range from the inclusion of hair conditioning aids in shampoos to post-shampoo application of hair conditioners, i.e., hair rinses. Hair rinses typically work by depositing a polymeric film or other material onto the hair. However, such solutions to a very prevalent problem have not been fully satisfactory. For one thing, hair rinses must be applied in a separate step following the shampooing, left on the hair for a length of time, and rinsed with fresh water. This, of course, is time consuming and is not convenient.

While shampoos, both antidandruff and regular, have been disclosed which contain conditioning aids, they have not been totally satisfactory for a variety of reasons. One problem relates to compatibility problems between good cleaning anionic surfactants and the fatty cationic agents which are good conditioning agents. This caused other surfactants such as nonionics, amphoterics and zwitterionics to be examined by workers in the field. Many of these efforts are reflected in patents issued in the conditioning shampoo area. See for example U.S. Patent 3,849,348, November 19, 1974 to Hewitt; U.S. Patent 3,990,991, November 9, 1961 to Gerstein; and U.S. Patent 3,822,312, July 2, 1974 to Sato.

The use of these other surfactants solved many of the compatibility problems but still did not provide complete answers in all areas. For instance cationic conditioners may not deliver the desired level of softness desired by users. Materials which can provide increased softness are silicones, both those which are soluble as well as insoluble in the shampoo matrix.

Silicones in shampoo compositions have been disclosed in a number of different publications. Such publications include U.S. Patent 2,826,551, March 11, 1958 to Geen; U.S. Patent 3,964,500, June 22, 1976 to Drakoff; U.S. Patent 4,364,837, December 21, 1982 to Pader; and British Patent 849,433, September 28, 1960 to Woolston. While these patents disclose silicone containing compositions, they also do not provide answers to all of the problems encountered in making a totally satisfactory product. One problem is that of keeping a dispersed, insoluble silicone material suspended and the total product stable.

One approach to stabilizing silicone containing shampoos has been to use certain long chain derivatives and other long chain derivatives. Such an approach is found in European Patent Application 0181773, published May 7, 1986, incorporated herein by reference.

This same approach has been used to suspend antidandruff agents such as zinc pyridinethione. An exemplary patent demonstrating this approach is U.S. Patent 4,470,892, September 11, 1984 to Winkler, incorporated herein by reference.

The present inventors have surprisingly found that when silicone and a pyridinethione salt are combined in systems as described above, the materials can both be suspended without increasing the amount of suspending agent, provide good hair conditioning and improved deposition of the pyridinethione salt.

It is an object of the present invention therefore, to provide a stable silicone containing conditioning lotion shampoo which also possesses superior antidandruff activity.

It is a further object of the present invention to provide silicone shampoo compositions which possess improved wet combing benefits.

These and other objects will become readily apparent from the detailed description which follows.

Unless otherwise indicated, all percentages and ratios herein are by weight.

## SUMMARY OF THE INVENTION

The present invention relates to lotion shampoo compositions comprising from about 5% to about 70% of a synthetic surfactant, about 0.01% to about 10.0% of an insoluble, nonvolatile silicone, about 0.5% to about 5.0% of a certain long chain derivative, from about 0.8% to about 4% of a pyridinethione salt and water. These as well as optional components are described in detail below.

DETAILED DESCRIPTION

The essential components of the present invention as well as optional components are given in the following paragraphs.

Surfactant

An essential component of the present compositions is a surfactant. The surfactant, which may be selected from any of a wide variety of synthetic anionic, amphoteric, zwitterionic and nonionic surfactants, is present at a level of from about 5% to about 70%, preferably from about 10% to about 30%, most preferably from about 10% to about 22%.

Synthetic anionic surfactants can be exemplified by the alkali metal salts of organic sulfuric reaction products having in their molecular structure an alkyl radical containing from 8 - 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$ - $C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and other known in the art.

Zwitterionic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

$$R^2 - \overset{\overset{(R^3)_x}{|}}{Y^{(+)}} - CH_2 - R^4 - Z^{(-)}$$

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to about 3 carbon atoms; X is 1 when Y is a sulfur atom and 2 when Y is a nitrogen or phosphorus atom; $R^4$ is an alkylene or hydroxyalkylene of from 1 to about 4 carbon atoms and Z is a radical selected from the group consisting of carboxylate, sulfonate, sulfate, phosphonate, and phosphate groups.

Examples include:

4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate:
3-[P,P-diethyl-P-3,6,9-trioxatetradexocylphosphonio]-2-hydroxypropane-1-phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-[P,P-dimethyl-P-dodecylphosphonio]-propane-1-phosphonate; and
5-[N,N-di(3-hydroxypropyl)-N-hexadecylammonio]-2-hydroxypentane-1-sulfate.

Other zwitterionics such as betaines are also useful in the present invention. Examples of betaines useful herein include the high alkyl betaines such as coco dimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl) carboxy methyl betaine, stearyl bis-(2-hydroxy-propyl) carboxymethyl betaine, oleyl dimethyl gamma-carboxypropyl betaine, lauryl bis-(2-hydroxypropyl) alpha-carboxyethyl betaine, etc. The sulfobetaines may be represented by coco dimethyl sulfopropyl betaine, stearyl dimethyl sulfopropyl betaine, lauryl dimethyl sulfoethyl betaine, lauryl bis-(2-hyroxy-ethyl) sulfopropyl betaine and the like; amido betaines and amidosulfobetaines, wherein the $RCONH(CH_2)_3$ radical is attached to the nitrogen atom of the betaine are also useful in this invention.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from

about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and the products sold under the trade name "Miranol" and described in U.S. Patent 2,528,378.

Nonionic surfactants, which are preferably used in combination with an anionic, amphoteric or zwitterionic surfactant, can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from about 5,000 to about 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.

4. Long chain tertiary amine oxides corresponding to the following general formula:

$R_1R_2R_3N \rightarrow O$

wherein $R_1$ contains an alkyl, alkenyl or monohydroxy alkyl radical of from about 8 to about 18 carbon atoms, from 0 to about 10 ethylene oxide moieties, and from 0 to 1 glyceryl moiety, and $R_2$ and $R_3$ contain from 1 to about 3 carbon atoms and from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi(2-hydroxyethyl) amine oxide, dimethyloctylamine oxide, dimethyl-decylamine oxide, dimethyltetradecylamine oxide, 3,6,9-trioxaheptadecyldiethylamine oxide, di(2-hydroxyethyl)-tetradecylamine oxide, 2-dode-coxyethyldimethylamine oxide, 3-dodecoxy-2-hydroxypropyldi(3-hydroxypropyl)amine oxide, dimethylhexadecylamine oxide.

5. Long chain tertiary phosphine oxides corresponding to the following general formula:

$RR'R''P \rightarrow O$

wherein R contains an alkyl, alkenyl or monohydroxyalkyl radical ranging from 8 to 18 carbon atoms in chain length, from 0 to about 10 ethylene oxide moieties and from 0 to 1 glyceryl moiety and R′ and R″ are each alkyl or monohydroxylalkyl groups containing from 1 to 3 carbon atoms. The arrow in the formula is a conventional representation of a semipolar bond. Examples of suitable phosphine oxides are: dodecyldimethylphosphine oxide, tetradecyldimethylphosphine oxide, tetradecylmethylethylphosphine oxide, 3,6,9,-trioxaoctadecyldimethylphosphine oxide, cetyldimethylphosphine oxide, 3-dodecoxy-2-hydroxypropyldi(2-hydroxyethyl) phosphine oxide, stearyldimethylphosphine oxide, cetylethylpropylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl)phosphine oxide, tetradecylmethyl-2-hydroxypropylphosphine oxide, oleyldimethylphosphine oxide, 2-hydroxydodecyldimethylphosphine oxide.

6. Long chain dialkyl sulfoxides containing one short chain alkyl or hydroxy alkyl radical of 1 to about 3 carbon atoms (usually methyl) and one long hydrophobic chain which contain alkyl, alkenyl, hydroxy alkyl, or keto alkyl radicals containing from about 8 to about 20 carbon atoms, from 0 to about 10 ethylene oxide moieties and from 0 to 1 glyceryl moiety. Examples include: octadecyl methyl sulfoxide, 2-ketotridecyl methyl sulfoxide, 3,6,9,-trioxaoctadecyl 2-hydroxyethyl sulfoxide, dodecyl methyl sulfoxide, oleyl 3-hydroxypropyl sulfoxide, tetradecyl methyl sulfoxide, 3-methoxytridecyl methyl sulfoxide, 3-hydroxytridecyl methyl sulfoxide, 3-hydroxy-4-dodecoxybutyl methyl sulfoxide.

Many additional nonsoap surfactants are described in McCUTCHEON'S, DETERGENTS and EMULSIFIERS, 1979 ANNUAL, published by Allured Publishing Corporation, which is incorporated herein by reference.

The above-mentioned surfactants can be used alone or in combination in the shampoo compositions of the present invention. The anionic surfactants, particularly the alkyl sulfates, the ethoxylated alkyl sulfates and mixtures thereof are preferred for use herein as well as the isethionates.

Non-Volatile Silicone Material

Silicone fluids are a suitable nonvolatile silicone that may be used in the present compositions.

The nonvolatile silicone fluid may be either a polyalkyl siloxane, a polyaryl siloxane, a polyalkylaryl siloxane or a polyether siloxane copolymer and is present at a level of from about 0.1% to about 10.00% preferably from about 0.5% to about 5.0%. Mixtures of these fluids may also be used and are preferred in certain executions. The dispersed silicone particles should also be insoluble in the shampoo matrix. This is the meaning of "insoluble" as used hereinbefore and hereinafter.

The essentially nonvolatile polyalkyl siloxane fluids that may be used include, for example, polydimethyl siloxanes with viscosities ranging from about 5 to 600,000 centistokes at 25° C. These siloxanes are available, for example, from the General Electric Company as the Viscasil series and from Dow Corning as the Dow Corning 200 series. The viscosity can be measured by means of a glass capillary viscometer as set forth in Dow Corning Corporate Test Method CTM0004, July 20, 1970. Preferably the viscosity ranges from about 350 centistokes to about 200,000 centistokes.

The essentially nonvolatile polyalkylaryl siloxane fluids that may be used include, for example, polymethylphenylsiloxanes having viscosities of about 15 to 30,000 centistokes at 25° C. These siloxanes are available, for example, from the General Electric Company as SF 1075 methyl phenyl fluid or from Dow Corning as 556 Cosmetic Grade Fluid.

The essentially nonvolatile polyether siloxane copolymer that may be used is, for example, a polypropylene oxide modified dimethylpolysiloxane (e.g., Dow Corning DC-1248) although ethylene oxide or mixtures of ethylene oxide and propylene oxide may also be used.

References disclosing suitable silicone fluids include the previously mentioned U.S. Patent 2,826,551 to Geen; U.S. Patent 3,964,500, June 22, 1976 to Drakoff; U.S. Patent 4,364,837 to Pader and British Patent 849,433 to Woolston. All of these patents are incorporated herein by reference. Also incorporated herein by reference is Silicon Compounds distributed by Petrarch Systems, Inc., 1984. This reference provides a very good listing of suitable silicone materials.

Another silicone material found especially useful in the present compositions to provide good dry combing is a silicone gum. Silicone gums described by Petrarch and others including U.S. Patent 4,152,416, May 1, 1979 to Spitzer, et al. and Noll, Walter, Chemistry and Technology of Silicones, New York: Academic Press 1968. Also describing silicone gums are General Electric Silicone Rubber Product Data Sheets SE 30, SE 33, SE 54 and SE 76. All of these described references are incorporated herein by reference. "Silicone gum" materials denote high molecular weight polydiorganosiloxanes having a mass molecular weight of from about 200,000 to about 1,000,000. Specific examples include polydimethylsiloxane, (polydimethylsiloxane) (methylvinylsiloxane) copolymer, poly(dimethylsiloxane) (diphenyl) (methylvinylsiloxane) copolymer and mixtures thereof.

Long Chain Derivative Suspending Agent

The suspending agent useful in the present compositions can be any of several long chain acyl derivative materials or mixtures of such materials. Included are ethylene glycol esters of fatty acids having from about 16 to about 22 carbon atoms. Preferred are the ethylene glycol stearates, both mono and distearate, but particularly the distearate containing less than about 7% of the mono stearate. Other suspending agents found useful are alkanol amides of fatty acids, having from about 16 to about 22 carbon atoms, preferably about 16 to 18 carbon atoms. Preferred alkanol amides are stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); glyceryl esters (e.g., glyceryl distearate) and long chain esters of long chain alkanol amides (e.g., stearamide DEA distearate, stearamide MEA stearate).

Still other suitable suspending agents are alkyl ($C_{16-22}$) dimethyl amine oxides such as stearyl dimethyl amine oxide. If the compositions contain an amine oxide or a long chain acyl derivative as a surfactant the suspending function could also be provided and additional suspending agent may not be needed if the level of those materials are at least the minimum level given below.

The suspending agent is present at a level of from about 0.50% to about 5.0%, preferably from about 0.5% to about 3.0%. The suspending agent serves to assist in suspending the silicone material and the pyridinethione salt and may give pearlescence to the product. Mixtures of suspending agents are also suitable for use in the compositions of this invention.

Pyridinethione Salt Crystals

Pyridinethione salts in crystal form are the antidandruff agents used in the present compositions. The crystals have a mean particle size in the range of from about 1μ to about 30μ. Such crystals are heavy metal, magnesium or aluminum salts of 1-hydroxy-2-pyridinethione which has the following structural formula in tautomeric form, the sulfur being attached to the No. 2 position in the pyridine ring.

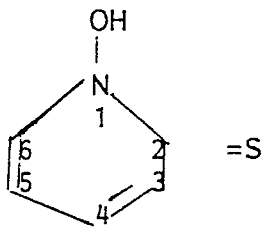

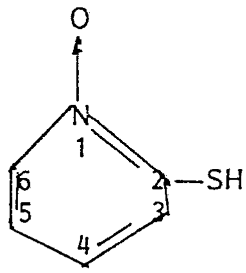

The metal salts represent substitution of the metal for the hydrogen of one of the tautomeric forms. Depending, of course, on the valence of the metal involved there may be more than one of the pyridinethione rings in the compound. Suitable heavy metals inlude zinc, tin, cadmium and zirconium.

The preferred pyridinethione salt crystals used in the present invention are predominantly flat platelets which have a mean sphericity less than about 0.65, preferably between about 0.20 and about 0.65 and a median particle size of at least about 2μ diameter, expressed as the median equivalent diameter of a sphere of equal volume. It is preferred that the mean individual particle size be not greater than about 25μ, measured on the same basis. The median diameters are on a mass basis with 50% of the mass of particles falling on either side of the value given.

The diameter of a sphere of equivalent volume for a particle can be determined by a variety of sedimentation techniques which are based on Stokes' Law for the settling velocity of a particle in a fluid. Such techniques are described in Stockham, J.D. and Fochtman, E.G., Particle Size Analysis,, Ann Arbor Science, 1978, incorporated herein by reference. The sphericity of a particle is also described by Stockham and Fochtman at page 113 as

$$Z = \left(\frac{d_n}{d_s}\right)^2$$

where $d_v$ is the diameter of a sphere of equivalent volume, supra, and $d_s$ is the diameter of a sphere of equivalent area. In the present invention

$$\text{the mean sphericity} = \left(\frac{\bar{d}_n}{\bar{d}_s}\right)^2 \quad \text{or}$$

surface area of spheres having equivalent volume distribution divided by the actual surface area of particles as measured.

A technique for determining actual surface area is shown in the examples using the BET technique described by Stockman and Fochtman at page 122.

The pyridinethione salt is used at a level of from about 0.8% to about 4%, preferably from about 1.0 to 2.0%.

Water

Water is the last essential component of the present invention and forms the remainder of the composition. It is generally present at a level of from about 20% to about 95%, preferably from about 60% to about 85%.

Optional Components

The shampoos herein can contain a variety of nonessential optional components suitable for rendering such compositions more acceptable. Such conventional optional ingredients are well known to those skilled in the art, e.g., preservatives such as benzyl alcohol, methyl paraben, propyl paraben and imidazolidinyl urea; cationic surfactants such as cetyl trimethyl ammonium chloride, lauryl trimethyl ammonium chloride, tricetyl

methyl ammonium chloride, stearyldimethyl benzyl ammonium chloride, and di(partially hydrogenated tallow) dimethylammonium chloride; thickeners and viscosity modifiers such as a diethanolamide of a long chain fatty acid (e.g., PEG 3 lauramide), block polymers of ethylene oxide and propylene oxide such as Pluronic F88 offered by BASF Wyandotte, sodium chloride, sodium sulfate, polyvinyl alcohol, and ethyl alcohol; pH adjusting agents such as citric acid, succinic acid, phosphoric acid, sodium hydroxide, sodium carbonate, etc.; perfumes; dyes; and, sequestering agents such as disodium ethylenediamine tetraacetate. Such agents generally are used individually at a level of from about 0.01% to about 10%, preferably from about 0.5% to about 5.0% by weight of the composition.

The pH of the present compositions is not critical and may be in the range of from 2 to about 10, preferably from about 4 to about 7. The compositions generally have a viscosity of from about 1,000 to about 12,000 at 25° C.

METHOD OF MANUFACTURE

One method for manufacturing the present composition is described below.

All ingredients except the pyridinethione salt are mixed together and heated to about 72° C. The mixture is mixed thoroughly for about 10 minutes at 72° C before pumping through a high shear mill and then through a heat exchanger to cool to about 27° C. The pyridinethione salt is then added at 27° C and it is thoroughly mixed.

The high shear mill is used to achieve adequate dispersion of the silicone fluid. This is achieved by having the average particle size of about 10μ or less.

In the cooling step, the acyl derivative is preferably crystallized into particles having an average particle size of about 10μ or less.

In an alternative process a part of the surfactant is not added until after the product has cooled. This may help control the crystallization of the acyl derivative.

INDUSTRIAL APPLICABILITY

The present compositions are used in a conventional manner for cleaning hair. From about 0.1g to about 10g of a composition is applied to hair that has been wetted, generally with water, worked through the hair and then rinsed out.

The following Examples further described and demonstrate the preferred embodiments within the scope of the present invention. The Examples are given solely for the purpose of illustration and are not to be construed as limitations of the present invention as many variations thereof are possible without departing from its spirit and scope.

EXAMPLES I - IV

The following compositions are representative of the present invention.

| | Weight % | | | |
|---|---|---|---|---|
| Component: | I | II | III | IV |
| Pynthione Zinc[1] | 1.0% | 1.0% | 1.0% | 1.0% |
| Ammonium Lauryl Sulfate | 15 | 15 | 13.5 | 13.5 |

| | | | | |
|---|---|---|---|---|
| Ammonium Laureth-3 Sulfate | 4.0 | 4.0 | 4.0 | 4.0 |
| Ammonium Xylene Sulfonate | 1.6 | 1.6 | 1.2 | 1.2 |
| Dimethicone[2] | 2.0 | 1.0 | 3.0 | 1.5 |
| Glycol Distearate | 2.0 | 2.0 | 2.0 | 2.0 |
| Cocamide MEA | 1.5 | 1.5 | 1.5 | 1.5 |
| Cetyl Alcohol | 0.63 | 0.63 | 0.42 | 0.42 |
| Tricetyl Ammonium Chloride | 0.50 | 0.50 | 0.50 | 0.50 |
| Sodium Hydroxide | 0.175 | 0.175 | 0.15 | 0.15 |
| Stearyl Alcohol | 0.270 | 0.27 | 0.18 | 0.18 |
| Sodium Chloride | 0.01 | 0.01 | 0.01 | 0.01 |
| Sodium Citrate | 0.0038 | 0.0038 | 0.05 | 0.05 |
| Preservative, Dye, Perfume and Water | q.s. to 100% | | | |
| pH | 4.6 | 4.6 | 4.6 | 4.6 |

[1]Zinc pyridinethione in platelet form having a mean sphericity of less than about 0.65 and a median particle size of about 18μ.

[2]Polydimethylsiloxane from General Electric having a viscosity of $10^6$ cps.

EXAMPLES V - VI

The following are two additional examples of the present invention.

| Component: | Weight % V | Weight % VI |
|---|---|---|
| Zinc pynthione[1] | 3.5 | 0.8 |
| Ammonium lauryl Sulfate | 13.5 | 13.5 |
| Ammonium Laureth-3 Sulfate | 4.0 | 4.0 |
| Dimethicone[2] | 4.0 | 1.0 |
| Glycol Distearate | 5.0 | 1.5 |
| Ammonium Xylenesulfonate | 1.2 | 1.2 |

| | | |
|---|---|---|
| Fragrance | 0.65 | 0.65 |
| Cocamide MEA | 1.50 | 1.50 |
| Tricetylmonium Chloride | 0.50 | 0.50 |
| Cetyl Alcohol | 0.42 | 0.63 |
| Stearyl Alcohol | 0.18 | 0.27 |
| Sodium Chloride | 0.1 | 0.1 |
| Sodium Hydroxide | 0.1 | 0.1 |
| Preservative | 0.000495 | 0.000495 |
| Sodium Citrate | 0.05 | 0.05 |
| Color | 0.1 | 0.1 |
| Water | q.s. 100% | q.s. 100% |

[1]As in Examples I-IV
[2]As in Examples I-IV

EXAMPLES VII - VIII

The following are additional compositions representative of the present invention.

| | Weight % | |
|---|---|---|
| Component: | VII | VIII |
| Ammonium Lauryl Sulfate | - | 3.06 |
| Ammonium $AE_3S$ | 8.78 | 11.32 |
| Ammonium Xylene Sulfonate | 0.20 | 0.20 |
| Cocamide MEA | 3.00 | 3.00 |
| Glycol Distearate | 3.00 | 3.00 |
| Zinc Pyrithione[1] | 1.00 | 1.00 |
| Fragrance | 0.65 | 0.65 |
| Cetyl Alcohol | 0.42 | 0.42 |
| Stearyl Alcohol | 0.18 | 0.18 |
| TCMAC | 0.48 | 0.48 |
| Silicone Gum Mixture[2] | 1.00 | 1.00 |
| Sodium Cocoyl Isethionate | 7.64 | 5.10 |
| pH Adjustment, Color, Preservative and Water | q.s. 100.% | 100.% |

[1]As in Examples I-IV
[2]As in Examples I-IV

## Claims

1. A shampoo composition comprising:
a) 5% to 70% by weight of a synthetic surfactant;
b) from 0.01% to 10% by weight of a dispersed, insoluble nonvolatile silicone;
c) from 0.5% to 5% by weight of a long chain ($C_{16}$-$C_{22}$) acyl derivative or a long chain ($C_{16}$-$C_{22}$) amine oxide;
d) from 0.8% to 4% by weight of a pyridinethione salt; and
e) water.

2. A shampoo composition according to Claim 1 wherein the surfactant is selected from anionic surfactants, zwitterionic surfactants, amphoteric surfactants and mixtures thereof.

3. A shampoo composition according to Claim 1 or 2 wherein the long chain acyl derivative or amine oxide is selected from ethylene glycol long chain esters, alkanol amides of long chain fatty acids, long chain esters of long chain fatty acids, glyceryl long chain esters, long chain esters of long chain alkanolamides, long chain alkyl dimethyl amine oxides and mixtures thereof.

4. A shampoo composition according to any of Claims 1 to 3 wherein the pyridinethione salt is in the form of platelets having a mean sphericity of less than about 0.65 and a median diameter of at least 2μ.

5. A shampoo composition according to any of Claims 1 to 4 wherein the nonvolatile silicone is selected from polydimethylsiloxanes having viscosities of from 5 to 200,000 centistokes at 25°C, polypropylene oxide modified dimethylsiloxanes and silicone gums.

6. A shampoo composition according to any of Claims 1 to 5 wherein the surfactant is selected from amphoteric surfactants, zwitterionic surfactants and mixtures thereof.

7. A shampoo composition according to any of Claims 1 to 5 wherein the surfactant is anionic.

8. A shampoo composition according to any of Claims 1 to 7 which in addition contains a quaternary ammonium compound.

9. A shampoo composition according to any of Claims 1 to 8 wherein the level of zinc pyridinethione is from 0.8 to 2.0% by weight.

10. A shampoo composition according to any of Claims 1 to 9 wherein the acyl derivative is ethylene glycol distearate.

11. A shampoo composition according to any of Claims 1 to 10 wherein the surfactant is a mixture of alkyl sulfate and ethoxylated alkyl sulfate surfactants.